(19) **Europäisches Patentamt**
**European Patent Office**
**Office européen des brevets**

(11) **EP 3 742 328 A1**

(12) **EUROPEAN PATENT APPLICATION**
published in accordance with Art. 153(4) EPC

(43) Date of publication:
**25.11.2020 Bulletin 2020/48**

(21) Application number: **18908483.3**

(22) Date of filing: **16.11.2018**

(51) Int Cl.:
**G06K 9/00** (2006.01)

(86) International application number:
**PCT/CN2018/115772**

(87) International publication number:
**WO 2019/169896 (12.09.2019 Gazette 2019/37)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **09.03.2018 CN 201810194220**

(71) Applicant: **South China University of Technology**
**Guangzhou, Guangdong 510640 (CN)**

(72) Inventors:
• **HUANG, Han**
**Guangzhou, Guangdong 510640 (CN)**
• **LI, Zilong**
**Guangzhou, Guangdong 510640 (CN)**
• **HAO, Zhifeng**
**Guangzhou, Guangdong 510640 (CN)**

(74) Representative: **Petraz, Gilberto Luigi et al**
**GLP S.r.l.**
**Viale Europa Unita, 171**
**33100 Udine (IT)**

(54) **FATIGUE STATE DETECTION METHOD BASED ON FACIAL FEATURE POINT POSITIONING**

(57) Disclosed is a fatigue detection method based on facial feature point positioning. A user aims a camera at a face, and during detection after an initialization stage, image of each frame inputted by a monitoring video is detected using a face detection and facial feature point positioning algorithm to obtain facial feature points. An eye-related area is determined according to the facial feature points of the user, and whether the user has closed eyes is determined according to a degree of change in a grayscale value of the eye-related area, and an alert concerning a fatigue level of the user is issued according to an eye closure condition in an adjacent frame. The present invention is simple to use, has a small amount of calculation, is accurate in determination, may be applied to a real-time environment, and effectively reminds the user to rest, thus ensuring life and property security of the user.

FIG. 1

**Description**

**TECHNICAL FIELD**

The present invention belongs to the technical field of fatigue detection, and more particularly, relates to a fatigue detection method based on facial feature point positioning.

**BACKGROUND**

Present common fatigue detection methods include: brain wave detection, facial state detection and physiological index detection. The present methods have the following defects: firstly, devices depending on physiological indexes such as brain waves are complicated and non-portable; and secondly, monitoring instruments are expensive and not suitable for practical application. However, the present invention has a fast processing speed and a high detection accuracy, and is able to ensure life and property security of a user based on a quick reminder when the user suffers from fatigue phenomenon.

**SUMMARY**

The present invention provides a fatigue detection algorithm based on facial feature point positioning which is directed to the defects in a current fatigue driving detection method. The present invention is intended to automatically detect a fatigue level of a user in real time. The detection is divided into two parts, one is to initialize the fatigue detection algorithm, and the other is to detect the fatigue level according to each frame inputted by a video.
The following technical solutions are employed in the present invention to achieve the above objective.
A fatigue detection method based on facial feature point positioning of the present invention includes the following steps:

(a) data entry stage: performing initialization data entry for initial fatigue detection by a user according to a prompt, and after a face is stabilized, reading in first M frames of a stabilized video as an initialization frame set;
(b) initialization stage: counting feature information of the face and eyes in images of all frames in the initialization frame set, and performing initialization task to determine a face position search range and a face size range;
(c) eye closure detection stage: performing eye closure detection on each frame image inputted by a camera through feature point positioning and binarization;
(d) fatigue detection stage: making a fatigue judgment according to an eye closure condition of a current frame and an eye closure condition of several previous frames, and making a reminder; and
(e) new stage: counting an elapsed time, taking N minutes as a time interval, saving five images selected according to a set time interval per minute, and updating a weight according to images of the M frames in the time interval by the end of the N minutes.

As a preferred technical solution, the step (a) includes the following steps:

(a-1) according to the prompt, placing the camera at a position closest to the user to ensure that a largest face detected by the camera is a face of the user, and inputting a video under a condition that an expression of the user is natural; and
(a-2) selecting a sample from images of every 10 frames in the inputted video, when a change in a center value of a face box in adjacent samples is less than 20% of an edge length of the face box, determining that a face state movement range is small, and at the moment, entering images of 25 consecutive frames as the initialization frame set.

As a preferred technical solution, the step (b) includes the following steps:

(b-1) traversing images of the M frames, determining a face detection rectangular box of the user as a largest face rectangular box detected in each frame according to prior knowledge that the user is closest to the camera, counting an average width $W_0$ and an average height $H_0$ thereof as well as an average grayscale value $GRAY\_FACE_0$ thereof, detecting the facial feature points of the face of the user in the first M frames, determining positions of left and right eye pupils of the user, and obtaining an average pupil distance accordingly;
(b-2) determining a radius $R_0$ of a round eye approximation area according to the average pupil distance of the user and a size of the face rectangular frame, calculating the grayscale value of the eyes in the approximation area, binarizing the round eye approximation area in the first M frames, a binarized threshold value being the $GRAY\_FACE_0$, and counting a binarized value, an average grayscale value of an eye area in the first M frames being an average

grayscale value $GRAY\_EYE_0$ of the eyes of the user; and

(b-3) configuring a lowest detection width of the face detection box as 0.7 Wo and a lowest detection height as 0.7 $H_0$, initializing a face detection search range SEARCH_RECT as being centered on a center of the face detection box of the user, with a width of 2 $W_0$ and a height of 2 $H_0$, and updating a grayscale value $GRAY\_EYE'$ of the eyes as $GRAY\_EYE_0$.

As a preferred technical solution, the step (c) includes the following steps:

(c-1) performing face detection in a rectangular range of SEARCH RECT, when no faces are detected, prompting abnormal face detection; and when faces are detected, selecting the largest face therein, and recording the largest face as a face rectangle $R_f(t)$ of the user at a present stage;

(c-2) counting an average grayscale value of a face rectangle Ft of the user at the present stage, and recording as $G_f(t)$;

(c-3) performing facial feature point positioning on the face Ft of the user at the present stage, and locating positions of left and right eye pupils, a nasal tip, and left and right corners of a mouth;

(c-4) counting and binarizing grayscale values in two round areas having a radius R by taking the left and right eye pupils as centers, a binarized threshold value being $G_f(t)$, and configuring the binarized average grayscale value of an eye area as $G_e(t)$; and

(c-5) when $\dfrac{G_e(t) - GRAY\_EYE'}{GRAY\_EYE'} > 0.5$, determining that the eyes are closed, and an eye closure function is C(t) = 1; otherwise, C (t) = 0.

As a preferred technical solution, the step (d) includes the following steps:

(d-1) configuring a time elapsed from a current time point to algorithm initialization as t frames, and a fatigue level of the user at the current time point as Tired (t); and

(d-2) determining the fatigue level of the user at the current time point using a one-dimensional weighting method:

$$Tired(t) = \sum_{i=t-3\,00}^{t} C(i) * TIRED\_COE^i, t > 300.$$

As a preferred technical solution, the step (e) includes the following steps:

(e-1) traversing images of the M frames for updating, counting an average width $W_u$ and an average height $H_u$ of the face of the user as well as an average grayscale value $GRAY\_FACE_u$ thereof, detecting the facial feature points of the face of the user in the first M frames, determining positions of left and right eye pupils of the user, and obtaining an average pupil distance by averaging;

(e-2) determining an updated radius $R_u$ of a round eye approximation area according to the average pupil distance of the user and a size of the face rectangular frame, calculating a grayscale value of the eyes in the approximation area, binarizing the round eye approximation area in the first M frames, a binarized threshold value being $GRAY\_FACE_u$, and counting a binarized average grayscale value of an eye area in the first M frames as the average grayscale value $GRAY\_EYE_u$ of the eyes of the user;

(e-3) updating a lowest detection width of a face detection frame of an algorithm as 0.7 $W_u$ and a lowest detection length as 0.7 $H_u$, and updating a face detection search range as being centered on a center of the face detection box of the user, with a width of 2 $W_u$ and a height of $2H_u$; and

(e-4) updating an average grayscale value $GRAY\_EYE'$ of the eye area as $GRAY\_EYE_u$.

As a preferred technical solution, the M is 25, and the N is 5.

Compared with the prior art, the present invention has the following advantages and beneficial effects.

1. The grayscale initialization of the face and the eyes and an update strategy in the present invention ensure that the present invention is able to adapt to an illumination change and has stronger robustness and accuracy.

2. The initialization task of the face search range in the present invention ensures that the present invention is able to avoid unnecessary calculation, thus greatly reducing a calculation time, and improving an operation efficiency.

3. A fatigue value weighting strategy in the present invention counts an eye closure time of the user within a time interval, thus avoiding an interference caused by quick blinking of the user in a short time, and having a stronger robustness.

4. Compared with a method for judging blinking by an aspect ratio of an eye fitting ellipse, the present invention has a higher accuracy and a lower computational complexity.

## BREIF DESCRIPTION OF THE DRAWINGS

FIG. 1 is an overall flow chart of a face detection method based on facial feature points of the present invention.
FIG. 2 is an illustration diagram of an eye approximation area obtained by the present invention according to facial feature point positioning.
FIG. 3 is a diagram showing a condition that eyes detected by the present invention are opened in a current frame.
FIG. 4 is a diagram showing a condition that the eyes detected by the present invention are closed in the current frame.

## DETAILED DESCRIPTION

The present invention is further described in detail with reference to the embodiments and the accompanying drawings, but the embodiments of the present invention are not limited thereto.

Embodiment

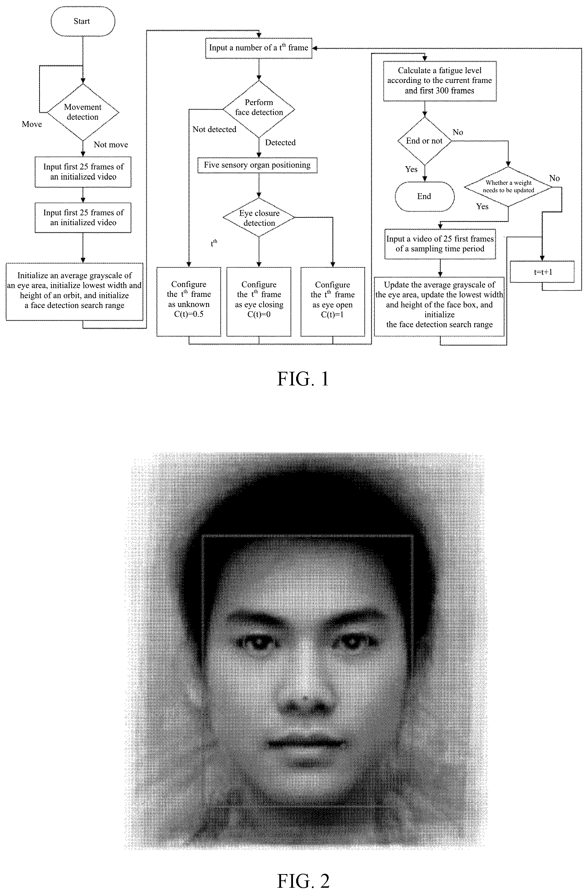

As shown in FIG. 1, a main flow of a face detection method based on deep learning includes the following steps.

(a) In a data entry stage, initialization data entry for initial fatigue detection is performed by a user according to a prompt. After a face is stabilized, top 25 frames of a stabilized video are read in by an algorithm as an initialization frame set.
The step (a) requires the user to perform the initialization data entry task under a quiet and stable condition. It should be noted that in a specific implementation process, a face detection and facial feature point positioning algorithm is not limited. However, the face detection should meet a real-time requirement and ensure that a detection speed is 25 frames/second and above, and the facial feature point positioning should ensure accurate positioning and meet the real-time requirement.
The step (a) mainly includes the following steps.

(a-1) According to the prompt of the algorithm, the camera is placed at a position closest to the user to ensure that a largest face detected by the camera is a face of the user, and a video is inputted under a condition that an expression of the user is natural.
(a-2) A sample is selected from images of every 10 frames in the inputted video, when a change in a center value of a face box in adjacent samples is less than 20% of an edge length of the face box, a face state movement range is determined to be small, and the initialization may be performed. At the moment, images of 25 consecutive frames are entered.

(b) In an algorithm initialization stage, feature information of the face and eyes in images of all frames in the initialization frame set is counted by the algorithm, and algorithm initialization is performed to determine a face position search range and a face size range.
The step (b) is mainly intended to perform the algorithm initialization, which mainly has two aspects, one is to determine a reference grayscale value, and the other is to determine the face search range. The determination of the reference grayscale value is intended to provide a judgment standard for eye closure detection for subsequent steps. The determination of the face search range is intended to reduce time consumption of the face detection, because in an actual application scene of driving, a change in a face size is relatively small, and a change in a face position is relatively small. We may use these two characteristics to reduce the time consumption of the face detection, and improve speeds of the face detection and the facial feature point positioning.
The step (b) mainly includes the following steps.

(b-1) Images of the 25 frames are traversed, and a face detection rectangular box of the user is determined as a largest face rectangular box detected in each frame according to prior knowledge that the user is closest to the camera. An average width $W_0$ and an average height $H_0$ thereof as well as an average grayscale value $GRAY\_FACE_0$ thereof are counted. The facial feature points of the face of the user in the first 25 frames are detected, positions of left and right eye pupils of the user are determined and recorded as $(\bar{x}_l, \bar{y}_l)$, $(\bar{x}_r, \bar{y}_r)$,, and an average pupil distance is obtained accordingly.

(b-2) A radius $R_0$ of a round eye approximation area is determined according to the average pupil distance of the user and a size of the face rectangular box, as shown in FIG. 2. In our experiment, a better value is determined as $R_0 = \left(\frac{7}{51}\right) * |\overline{x_r} - \overline{x_l}|$ , and the grayscale value of the eyes in the approximation area (with $(\overline{x_l}, \overline{y_l})$ and $(\overline{x_r}, \overline{y_r})$ used as centers, and $R_0$ used as a radius) is calculated. The round eye approximation area in the first 25 frames is binarized, a binarized threshold value is the $GRAY\_FACE_0$, and a binarized average grayscale value of an eye area in the first 25 frames is counted as an average grayscale value $GRAY\_EYE_0$ of the eyes of the user.

(b-3) A lowest detection width of the face detection box of the algorithm is configured as 0.7Wo and a lowest detection height is configured as $0.7H_0$. A face detection search range SEARCH_RECT is initiated as being centered on a center of the face detection box of the user, with a width of 2Wo and a height of $2H_0$. A grayscale value GRAY_EYE' of the eyes is updated as $GRAY\_EYE_0$.

(c) In an eye closure detection stage, eye closure detection is performed by the algorithm on each frame of image inputted by a camera through feature point positioning and binarization.

The step (c) is intended to perform the face detection with a specified lowest size in the above specified face detection area by the algorithm, mark the facial feature points, and determine whether the eyes are closed according to a change in the grayscale value in the round eye approximation area.

The step (c) includes the following steps.

(c-1) Face detection is performed in a rectangular range of SEARCH RECT, when no faces are detected, abnormal face detection is prompted; and when the faces are detected, the largest face therein is selected and recorded as a face rectangle $R_f(t)$ of the user at the stage.

(c-2) An average grayscale value of a face rectangle Ft of the user at the stage is counted, and recorded as $G_f(t)$.

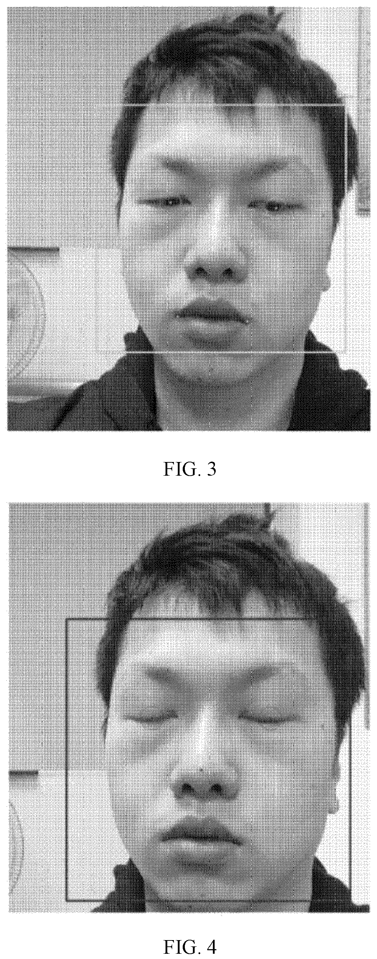

(c-3) Facial feature point positioning is performed on the face Ft of the user at the stage, and positions of left and right eye pupils, a nasal tip, and left and right corners of a mouth are located, as shown in FIG. 3 and FIG. 4.

(c-4) Grayscale values in two round areas having a radius R by taking the left and right eye pupils as centers are counted and binarized, a binarized threshold value is $G_f(t)$, and a binarized average grayscale value of an eye area is configured as $G_e(t)$.

(c-5) When $\frac{GRAY\_EYE - G_e(t)}{GRAY\_EYE} > 0.5$ , the eyes may be determined to be closed, and an eye closure function is C(t) = 1; otherwise, C (t) = 0.

(d) In adjacent frame fatigue detection stage, a fatigue judgment is made according to an eye closure condition of a current frame and an eye closure condition of several previous frames, and a reminder is made.

The step d is mainly intended to determine the fatigue level of the user according to eye-opening and eye closure conditions in adjacent time interval.

The step (d) includes the following steps.

(d-1) A time elapsed from a current time point to algorithm initialization is configured as t frames, and a fatigue level of the user at the current time point is configured as Tired (t).

(d-2) The fatigue level of the user at the current time point is determined using a one-dimensional weighting method.

$$Tired(t) = \sum_{i=t-3\,00}^{t} C(i) * TIRED\_COE^i, t > 300$$ .

(e) In a weight update stage, an elapsed time is counted by the algorithm, five minutes are taken as a time interval, images at a 0th second, a 12th second, a 24th second, a 36th second and a 48th second in each minute are saved, and a weight is updated according to images of the 25 frames in the time interval by the end of the five minutes.

The step (e) is mainly intended to update the weight to inhibit an influence of an illumination change on the fatigue detection.

The step (e) includes the following steps.

(e-1) Images of the 25 frames for updating are traversed, an average width $W_u$ and an average height $H_u$ of the face of the user as well as an average grayscale value $GRAY\_FACE_u$ thereof are counted. The facial feature points of the face of the user in the first 25 frames are detected, positions of left and right eye pupils of the user are determined, and an average pupil distance is obtained by averaging.

(e-2) An updated radius $R_u$ of a round eye approximation area is determined according to the average pupil distance of the user and a size of the face rectangular box, and a grayscale value of the eyes in the approximation area is calculated. The round eye approximation area in the first 25 frames is binarized, and a binarized threshold value is $GRAY\_FACE_u$. A binarized average grayscale value of a eye area in the first 25 frames is counted as an average grayscale value $GRAY\_EYE_u$ of the eyes of the user.

(e-3) A lowest detection width of the face detection box of the algorithm is updated as $0.7W_u$ and a lowest detection length is updated as $0.7H_u$. A face detection search range is updated as being centered on a center of the face detection box of the user, with a width of $2W_u$ and a height of $2H_u$.

(e-4) An average grayscale value $GRAY\_EYE'$ of the eye area is updated as $GRAY\_EYE_u$.

The above embodiments are the preferred embodiments of the present invention, but the embodiments of the present invention are not limited by the above embodiments. Any other changes, modifications, substitutions, combinations, and simplifications made without departing from the spirit and principle of the present invention should be equivalent substitute modes, and shall be included in the range of protection of the present invention.

**Claims**

1.  A fatigue detection method based on facial feature point positioning, **characterized in that**, the method comprises the following steps:

    (a) data entry stage: performing initialization data entry for initial fatigue detection by a user according to a prompt, and after a face is stabilized, reading in first M frames of a stabilized video as an initialization frame set;
    (b) initialization stage: counting feature information of the face and eyes in images of all frames in the initialization frame set, and performing initialization task to determine a face position search range and a face size range;
    (c) eye closure detection stage: performing eye closure detection on image of each frame inputted by a camera through feature point positioning and binarization;
    (d) fatigue detection stage: making a fatigue judgment according to an eye closure condition of a current frame and an eye closure condition of several previous frames, and making a reminder; and
    (e) new stage: counting an elapsed time, taking N minutes as a time interval, saving five images selected according to a set time interval per minute, and updating a weight according to images of the M frames in the time interval by the end of the N minutes.

2.  The fatigue detection method based on facial feature point positioning according to claim 1, **characterized in that**, the step (a) comprises the following steps:

    (a-1) according to the prompt, placing the camera at a position closest to the user to ensure that a largest face detected by the camera is a face of the user, and inputting a video under a condition that an expression of the user is natural; and
    (a-2) selecting a sample from images of every 10 frames in the inputted video, when a change in a center value of a face box in adjacent samples is less than 20% of an edge length of the face box, determining that a face state movement range is small, and at the moment, entering images of 25 consecutive frames as the initialization frame set.

3.  The fatigue detection method based on facial feature point positioning according to claim 1, **characterized in that**, the step (b) comprises the following steps:

    (b-1) traversing images of the M frames, determining a face detection rectangular box of the user as a largest face rectangular box detected in each frame according to prior knowledge that the user is closest to the camera, counting an average width $W_0$ and an average height $H_0$ thereof as well as an average grayscale value $GRAY\_FACE_0$ thereof, detecting the facial feature points of the face of the user in the first M frames, determining positions of left and right eye pupils of the user, and obtaining an average pupil distance accordingly;
    (b-2) determining a radius $R_0$ of a round eye approximation area according to the average pupil distance of the user and a size of the face rectangular box, calculating the grayscale value of the eyes in the approximation area, binarizing the round eye approximation area in the first M frames, a binarized threshold value being the $GRAY\_FACE_0$, and counting a binarized value, an average grayscale value of an eye area in the first M frames being an average grayscale value $GRAY\_EYE_0$ of the eyes of the user; and

(b-3) configuring a lowest detection width of the face detection box as 0.7Wo and a lowest detection height as $0.7H_0$, initializing a face detection search range SEARCH_RECT as being centered on a center of the face detection box of the user, with a width of $2W_0$ and a height of $2H_0$, and updating a grayscale value GRAY_EYE' of the eyes as $GRAY\_EYE_0$.

4. The fatigue detection method based on facial feature point positioning according to claim 1, **characterized in that**, the step (c) comprises the following steps:

(c-1) performing face detection in a rectangular range of SEARCH RECT, when no faces are detected, prompting abnormal face detection; and when the faces are detected, selecting the largest face therein, and recording the largest face as a face rectangle $R_f(t)$ of the user at the stage;

(c-2) counting an average grayscale value of a face rectangle Ft of the user at the stage, and recording as $G_f(t)$;

(c-3) performing facial feature point positioning on the face Ft of the user at the stage, and locating positions of left and right eye pupils, a nasal tip, and left and right corners of a mouth;

(c-4) counting and binarizing grayscale values in two round areas having a radius R by taking the left and right eye pupils as centers, a binarized threshold value being $G_f(t)$, and configuring the binarized average grayscale value of an eye area as $G_e(t)$; and

(c-5) when $\dfrac{G_e(t)-GRAY\_EYE'}{GRAY\_EYE'} > 0.5$ , determining that the eyes are closed, and an eye closure function is C(t) = 1; otherwise, C (t) = 0.

5. The fatigue detection method based on facial feature point positioning according to claim 1, **characterized in that**, the step (d) comprises the following steps:

(d-1) configuring a time elapsed from a current time point to algorithm initialization as t frames, and a fatigue level of the user at the current time point as Tired (t); and

(d-2) determining the fatigue level of the user at the current time point using a one-dimensional weighting method.

$$Tired(t) = \sum_{i=t-3\ 00}^{t} C(i) * TIRED\_COE^i, t > 300$$

6. The fatigue detection method based on facial feature point positioning according to claim 1, **characterized in that**, the step (e) comprises the following steps:

(e-1) traversing images of the M frames for updating, counting an average width $W_u$ and an average height $H_u$ of the face of the user as well as an average grayscale value $GRAY\_FACE_u$ thereof, detecting the facial feature points of the face of the user in the first M frames, determining positions of left and right eye pupils of the user, and obtaining an average pupil distance by averaging;

(e-2) determining an updated radius $R_u$ of a round eye approximation area according to the average pupil distance of the user and a size of the face rectangular box, calculating a grayscale value of the eyes in the approximation area, binarizing the round eye approximation area in the first M frames, a binarized threshold value being $GRAY\_FACE_u$, and counting a binarized average grayscale value of an eye area in the first M frames as an average grayscale value $GRAY\_EYE_u$ of the eyes of the user; and

(e-3) updating a lowest detection width of a face detection box of an algorithm as $0.7W_u$ and a lowest detection length as $0.7H_u$, and updating a face detection search range as being centered on a center of the face detection box of the user, with a width of $2W_u$ and a height of $2H_u$; and

(e-4) updating an average grayscale value GRAY_EYE' of the eye area as $GRAY\_EYE_u$.

7. The fatigue detection method based on facial feature point positioning according to any one of claims 1 to 6, **characterized in that**, the M is 25, and the N is 5.

FIG. 1

FIG. 2

FIG. 3

FIG. 4

## INTERNATIONAL SEARCH REPORT

| International application No. |
| --- |
| **PCT/CN2018/115772** |

**A. CLASSIFICATION OF SUBJECT MATTER**

G06K 9/00(2006.01)i

According to International Patent Classification (IPC) or to both national classification and IPC

**B. FIELDS SEARCHED**

Minimum documentation searched (classification system followed by classification symbols)

G06K

Documentation searched other than minimum documentation to the extent that such documents are included in the fields searched

Electronic data base consulted during the international search (name of data base and, where practicable, search terms used)

CNPAT, WPI, EPODOC, CNKI: 华南理工大学, 黄翰, 脸, 眼, 睁, 闭, 特征点, 定位, 疲劳, 检测, 二值, 阈值, 灰度, 平均, fatigue, face? , eye? , close, average, detect+, threshold

**C. DOCUMENTS CONSIDERED TO BE RELEVANT**

| Category* | Citation of document, with indication, where appropriate, of the relevant passages | Relevant to claim No. |
| --- | --- | --- |
| PX | CN 108742656 A (SOUTH CHINA UNIVERSITY OF TECHNOLOGY) 06 November 2018 (2018-11-06) claims 1-7, description, paragraphs [0046]-[0078], and figures 1-4 | 1-7 |
| Y | CN 104809482 A (NANJING UNIVERSITY) 29 July 2015 (2015-07-29) description, paragraphs [0031]-[0075], and figures 1-4 | 1, 2, 5, 7 |
| Y | CN 107563346 A (NANJING OAK TRANSPORT INTERNET TECHNOLOGY CO., LTD.) 09 January 2018 (2018-01-09) description, paragraphs [0037]-[0056], and figure 1 | 1, 2, 5, 7 |
| A | CN 106250801 A (BAICYINXIANG AUTOMOBILE CO., LTD.) 21 December 2016 (2016-12-21) entire document | 1-7 |
| A | WO 0209025 A1 (SEEING MACHINES PTY. LTD.) 31 January 2002 (2002-01-31) entire document | 1-7 |
| A | CN 106846734 A (NANJING UNIVERSITY OF SCIENCE AND TECHNOLOGY) 13 June 2017 (2017-06-13) entire document | 1-7 |

☐ Further documents are listed in the continuation of Box C.      ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **17 January 2019** | **02 February 2019** |

| Name and mailing address of the ISA/CN | Authorized officer |
| --- | --- |
| **National Intellectual Property Administration, PRC (ISA/ CN)** **No. 6, Xitucheng Road, Jimenqiao Haidian District, Beijing 100088** **China** | |
| Facsimile No. **(86-10)62019451** | Telephone No. |

Form PCT/ISA/210 (second sheet) (January 2015)

**INTERNATIONAL SEARCH REPORT**
Information on patent family members

| International application No. |
| --- |
| **PCT/CN2018/115772** |

| Patent document cited in search report | | | Publication date (day/month/year) | Patent family member(s) | | | Publication date (day/month/year) |
| --- | --- | --- | --- | --- | --- | --- | --- |
| CN | 108742656 | A | 06 November 2018 | None | | | |
| CN | 104809482 | A | 29 July 2015 | None | | | |
| CN | 107563346 | A | 09 January 2018 | None | | | |
| CN | 106250801 | A | 21 December 2016 | None | | | |
| WO | 0209025 | A1 | 31 January 2002 | JP | 5016175 | B2 | 05 September 2012 |
| | | | | AU | PQ896000 | A0 | 17 August 2000 |
| | | | | DE | 60140067 | D1 | 12 November 2009 |
| | | | | JP | 2004504684 | A | 12 February 2004 |
| | | | | AU | PQ896000 | D0 | 17 August 2000 |
| | | | | EP | 1320830 | B1 | 30 September 2009 |
| | | | | US | 2003169907 | A1 | 11 September 2003 |
| | | | | EP | 1320830 | A1 | 25 June 2003 |
| | | | | US | 7043056 | B2 | 09 May 2006 |
| CN | 106846734 | A | 13 June 2017 | None | | | |

Form PCT/ISA/210 (patent family annex) (January 2015)